# EUROPEAN PATENT APPLICATION

(11) **EP 4 238 559 A1**
(43) Date of publication of application: **06.09.2023**
(21) Application number: 22305250.7
(22) Date of filing: 04.03.2022
(51) Int. Cl.: A61K 31/167, A61K 31/198, A61P 29/00

(54) **COMBINATIONS OF ACETAMINOPHEN AND N-ACETYL CYSTEINE FOR THE TREATMENT OF PAIN AND FEVER**

(71) Applicant: Centre Hospitalier Universitaire de Caen Normandie, 14000 Caen (FR)
(72) Inventor: CHRETIEN, Basile, 14033 CAEN (FR); DOLLADILLE, Charles, 14033 CAEN (FR)
(74) Representative: Regimbeau

(57) **Abstract**

The invention relates to a combination of acetaminophen and N-acetyl cysteine for its use in preventing and/or treating fever and/or pain, wherein:
- the administered daily dose of acetaminophen is superior to 4000 mg per day for an adult, or is superior to 60 mg per weight kilo per day for a child, and
- acetaminophen and N-acetyl cysteine are administered to said adult or child according to a ratio in weight of acetaminophen / N-acetyl cysteine comprised between 1 and 10.

## Description

### FIELD OF THE INVENTION

The present invention relates to compositions comprising a combination of active compounds for the treatment and/or prevention of pain and/or fever in mammals, including humans, both adults and children.

One of the compounds in the combination is acetaminophen, which presents a hepatic toxicity at high doses.

The present invention relates to compositions adapted for the administration of a high dose of acetaminophen, for preventing and/or treating pain and/or fever.

### BACKGROUND OF THE INVENTION

The improvement of pain management is a current therapeutic issue. In this regard, the current trend is to reduce the use of non-steroidal anti-inflammatory drugs (NSAIDs) and opiates, which have numerous contraindications.

Acetaminophen, also known as paracetamol or N-acetyl-p-aminophenol, is a reference drug for the treatment of fever and/or pain, at all ages and in many circumstances. It is the most commonly used medication for pain and fever, in both the United States and Europe. It is on the World Health Organization's List of Essential Medicines. Acetaminophen is available over the counter, without need of medical prescription.

Acetaminophen poisoning is the foremost cause of acute liver failure in Western countries, and accounts for most drug overdoses.

The current recommended maximum dose for adults is 4 grams per day, divided into doses of 1 gram maximum. This maximum dose is limited by the toxicity, mainly hepatic, that can occur at higher doses. However, the pharmacokinetics of acetaminophen at 4 grams per day expose it to periods of ineffectiveness. During these periods, fever cannot be relieved without the use of other medications, such as non-steroidal anti-inflammatory drugs. Unfortunately, these other drugs have a much poorer side effect profile than acetaminophen, and are usually not used in the context of infections, as they can lead to complications and even death.

Studies have shown that increasing the administered dose of acetaminophen actually increases its effectiveness. The challenge of prescribing acetaminophen in high doses, over a few days or more, while avoiding its toxicity, is therefore important and has no solution at the present time.

N-acetyl cysteine (NAC) is the reference antidote for acetaminophen poisoning. It allows to recharge the glutathione stock and to oppose N-acetyl-para-benzoquinone-imine, a toxic metabolite of acetaminophen (Ramlawi *et al*., 2013). Its mode of action does not interfere with the therapeutic efficacy of acetaminophen.

It has been previously proposed to combine acetaminophen and N-acetyl cysteine. Nevertheless, the aim of these combinations was either to use the mucolytic properties of NAC (for example in the products Fluimucil^{®} and Sistenol), either to treat overdose consequences of unintentional high consumption of acetaminophen, with a subsequent administration of NAC.

In this goal of treating acetaminophen poisoning, the patent application US 2008/0139654 discloses the administration of NAC to poisoned patients, and demonstrates the effectiveness of the approach with good recovery of at least one patient. This patent application also proposes dosing units comprising combinations of acetaminophen and NAC, having reduced hepatotoxicity.

The international application WO 02/078627 also relates to the combination of acetaminophen and NAC, NAC being used for reducing the toxicity of acetaminophen. Dosing units comprising the combination, orally administrable, are also disclosed.

However, no combination of acetaminophen and NAC has been proposed to date with the aim of increasing the daily dosage of administered acetaminophen through this association, in order to better manage fever and/or pain.

The patent FR 2,744,917 discloses the administration of acetaminophen in amounts greater than those normally used, in combination with NAC. However, these "greater amounts" are defined as greater than 3 grams per day for an adult.

It is indeed not an obvious practice for physicians or users to administrate / consume more than 4 grams of acetaminophen per day for an adult, as no study has demonstrated the effectiveness of such an approach for treating and/or preventing pain and/or fever.

Thus, even if both drugs exist on the market today, they have never been combined for the prevention and/or treatment of fever and/or pain by prescribers/users, in the specific doses and ratio of the claimed invention.

### SUMMARY OF THE INVENTION

The present invention relates to a combination of acetaminophen and N-acetyl cysteine for its use in preventing and/or treating fever and/or pain, wherein:
- the administered daily dose of acetaminophen is superior to 4000 mg per day for an adult, or is superior to 60 mg per weight kilo per day for a child, and
- acetaminophen and N-acetyl cysteine are administered to said adult or child according to a ratio in weight of acetaminophen / N-acetyl cysteine comprised between 1 and 10.

The present invention also relates to dosing units comprising, in a pharmaceutical formulation, a combination of acetaminophen and N-acetyl cysteine for its use in preventing and/or treating fever and/or pain, wherein:
- the administered daily dose of acetaminophen is superior to 4000 mg per day for an adult, or is superior to 60 mg per weight kilo per day for a child, and
- acetaminophen and N-acetyl cysteine are administered to said adult or child according to a ratio in weight of acetaminophen / N-acetyl cysteine comprised between 1 and 10.

The present invention also concerns a pharmaceutical kit comprising dosing units as defined above.

### DETAILED DESCRIPTION OF EMBODIMENTS OF THE INVENTION

Unless stated otherwise, the following terms and phrases as used herein are intended to have the following meanings.

Acetaminophen, CAS number 103-90-2, is an analgesic and antipyretic drug. It can be administered orally, rectally or intravenously. It is also referred to as "APAP".

Acetaminophen's metabolites are known to act to deplete cysteine/glutathione that helps to protect cells against oxidative damages, and to cause a variety of drug toxicity symptoms. It is well established that large acetaminophen overdose causes hepatotoxicity, and in some cases nephrotoxicity, in humans and animals.

In normal doses, acetaminophen is rapidly absorbed from the stomach and small intestine and is normally metabolized by conjugation in the liver into nontoxic agents, which are then eliminated in the urine.

When maximal daily doses are exceeded, the main pathways of metabolism become saturated. Excess acetaminophen is metabolized in the liver into a toxic compound designated as N-acetyl-p-benzoquinone-Imine (NAPQI). In usual dosages, this NAPQI is rapidly conjugated with glutathione but under conditions of excessive NAPQI formation or reduced glutathione content, NAPQI will bind to vital proteins and the lipid bilayer of hepatocytes. This results in hepatocellular death and subsequent centrilobular liver necrosis.

A known method of treatment for acetaminophen overdose is the administration of sulfhydryl compounds. L-methionine, L-cysteine, N-acetyl cysteine (L- enantiomer or racemate mixture) are known to have a protective action against acetaminophen toxicity. N-acetyl cysteine is the only pharmacological agent currently accepted for use in the treatment of acetaminophen intoxication.

N-acetyl cysteine, here also referred to as "NAC", CAS number 616-91-1, is a drug used to loosen thick mucus and to treat acetaminophen overdose. It can be administered orally, nasally, by ocular route, or intravenously.

N-acetyl cysteine is a prodrug to L-cysteine, a precursor to the antioxidant glutathione. Hence administration of NAC replenishes glutathione stores and counteracts the toxic effects of acetaminophen.

In the sense of the invention, NAC includes all stereoisomer formulations, in particular L-enantiomer formulation, D - enantiomer formulation, and a mixture comprising any ratio of L/D.

The present invention aims at administering high doses of acetaminophen for preventing and/or treating pain and/or fever in mammals, in particular in human beings.

Usually administered daily doses are of 4000 mg maximum for an adult, and up to 60 mg per weight kilo per day for a child.

In the sense of the invention, an adult designates a human being aged of at least 18 years. A child designates a human being aged of 1 day to 18 years. The term "child" includes newborns, infants and young children.

Children medicines are often presented under the form of syrup for an easier consumption and an easier dosing. Indeed, the doses are usually determined in function of the weight of the child, instead of its age. Children drugs are furnished with a chart presenting correspondence of weight and doses and a graduated dosing cup.

Examples of acetaminophen formulations adapted for infants are the Efferalganmed^{®} 80 mg (powder or suppository) or the injectable solution BBRAUN^{®}.

The combination and its administration mode according to the invention is used for preventing and/or treating fever and/or pain in mammals, preferably in human beings, also designated in the present application as patients.

In the sense of the invention, the term "preventing" or "prevention" means reducing the risk of occurrence or slowing down the occurrence of a given biological phenomenon, i.e., according to the present invention, the occurrence of fever and/or pain in a patient.

The term "treating" or "treatment" means reducing the intensity of the pain and/or fever symptoms. Ideally, a complete treatment of the fever significates that the body temperature of the patient returns to its baseline temperature, and a complete treatment of pain is reached when the patient does not feel any pain. Nevertheless, the term "treatment" also refers to the partial reduction of symptoms, for example a decrease of the body temperature and/or a reduction of the pain experienced by the patient, after administration of the combination. Means for measuring fever and/or pain are well known by the person of the art, and include thermometer and numerical rating scale for pain.

In a first embodiment of the invention, the combination is for its use in preventing fever and pain.

In a second embodiment of the invention, the combination is for its use in preventing fever.

In a third embodiment of the invention, the combination is for its use in preventing pain.

In a fourth embodiment of the invention, the combination is for its use in treating fever and pain.

In a fifth embodiment of the invention, the combination is for its use in treating pain.

In a sixth embodiment of the invention, the combination is for its use in treating fever.

In a seventh embodiment of the invention, the combination is for its use in preventing and treating fever and pain.

In a eighth embodiment of the invention, the combination is for its use in preventing and treating fever.

In a nineth embodiment of the invention, the combination is for its use in preventing and treating pain.

The present invention concerns a combination of acetaminophen and N-acetyl cysteine for its use in preventing and/or treating fever and/or pain, wherein:
- the administered daily dose of acetaminophen is superior to 4000 mg per day for an adult, or is superior to 60 mg per weight kilo per day for a child, and
- acetaminophen and N-acetyl cysteine are administered to said adult or child according to a ratio in weight of acetaminophen / N-acetyl cysteine comprised between 1 and 10.

In the sense of the invention, the "administered daily dose" means the amount of acetaminophen, in weight, administered during a period of one day, that is to say 24 hours, to one patient.

This daily dose is usually administered in one, two, three, four, five, six, seven, eight, nine, ten, eleven or twelve intakes during the period of 24 hours. It is better to respect a certain period of time between two intakes; in particular for acetaminophen, health providers advise to respect a time interval of at least four hours between two intakes, but in the context of the present invention, said interval of time may be decreased to two hours between two intakes.

Unless otherwise mentioned, in the present application, the values at the limits of the intervals are included in said intervals.

In an embodiment, the administered daily dose of acetaminophen is comprised between 4000 mg and 10000 mg per day for an adult, preferably between 5000 mg and 10000 mg per day for an adult.

In particular, the administered daily dose of acetaminophen is comprised between 5000 mg and 9000 mg, between 6000 mg and 8000 mg, or between 6000 mg and 10000 mg per day for an adult.

In particular, the administered daily dose is of at least 4100, 4200, 4300, 4400, or 4500 mg per day; or is of at least 5500, 6000, 6500, 7000, 7500, 8000, 8500, 9000, 9500 or 10000 mg per day for an adult.

In another embodiment, when the patient is a child, the administered daily dose of acetaminophen is comprised between 60 mg and 120 mg per weight kilo per day, in particular is comprised between 75 mg and 120 mg per weight kilo per day.

In particular, the administered daily dose of acetaminophen for a child is comprised between 70 mg and 120 mg, between 75 mg and 110 mg, or between 80 and 100 mg per weight kilo per day.

In particular, the administered daily dose is of at least 65, 70, 75, 80, 85, 90, 95, 100, 110 or 120 mg per weight kilo per day.

### Ratio APAP/NAC

The combination also presents a specific ratio of each active administered component, acetaminophen and N-acetyl cysteine.

In the sense of the invention, the term "ratio in weight" designates the quantity, in dry weight, of acetaminophen administered, divided by the quantity, in dry weight, of N-acetyl cysteine administered to the same patient, over a certain period of time. In particular, said period of time is 24 hours. This period of time might also be 12 hours, 6 hours, 4 hours, 2 hours, or 1 hour.

The ratio in weight of both components of the combination also refers to the respective proportion in weight of each component in a dosing unit comprising both components.

In a particular embodiment, in the combination for its use according to the invention, the ratio in weight of acetaminophen / N-acetyl cysteine (APAP/NAC) is comprised between 2 to 10, or is comprised between 3 to 9, or between 4 to 9, or between 5 to 9, or between 5 and 8, or between 6 and 8, the values at the limits of intervals being included.

In particular, the ratio APAP/NAC is equal to 5, 6, 7, 8 or 9.

### Modes of administration

The combination for its use according to the invention is composed of at least two active components, acetaminophen and NAC.

In a particular embodiment of the invention, both active components of the combination are administered simultaneously.

According to this embodiment, both active components may be administered under the form of one dosing unit comprising both active components; or under the form of two dosing units, each of them comprising one active component.

In another embodiment of the invention, the two components of the combination are administered sequentially.

According to this embodiment, the active components are administered at two different times to the same patient.

A time period between the administration of both components may be for example 1, 2, 3, 4, 5 or 6 hours. A longer time period of 24 hours may also be observed between administration of a first component (for example, acetaminophen) and a second component (for example NAC).

The combination for its use according to the invention may be administered for any period decided by the health provider.

In particular, the combination is administered for a period of at least one day, or at least two days, at least three days, at least four days, at least five days, at least six days, at least seven days, at least eight days, at least nine days, or even for ten days.

The present invention also concerns a dosing unit comprising, in a pharmaceutical formulation, a combination for its use according to the invention, that is to say for its use in preventing and/or treating fever and/or pain, wherein:
- the administered daily dose of acetaminophen is superior to 4000 mg per day for an adult, or is superior to 60 mg per weight kilo per day for a child, and
- acetaminophen and N-acetyl cysteine are administered to said adult or child according to a ratio in weight of acetaminophen / N-acetyl cysteine comprised between 1 and 10.

The terms "pharmaceutical formulation" designate a formulation that is compatible for the route of administration to a human being under consideration, that is to say that is non toxic and that is well tolerated by the human body.

In a first embodiment, the dosing unit is adapted for an administration to an adult.

For example, a dosing unit for adult comprises 500 to 1000 mg of acetaminophen and 50 to 1000 mg of N-acetyl cysteine, with a ratio in weight of acetaminophen/N-acetyl cysteine comprised between 4 and 10, preferably between 5 and 9.

More preferably, a dosing unit for adult comprises 500 to 1000 mg of acetaminophen and 60 to 250 mg of N-acetyl cysteine, with a ratio in weight of acetaminophen/N-acetyl cysteine comprised between 4 and 10, preferably between 5 and 9.

Examples of proportions of both components APAP and NAC in said dosing units are presented in table 1 below.

**Table 1. Example of dosing units for adults**

| Dosing unit n° | Acetaminophen (APAP) (mg) | N-acetyl cysteine (NAC) (mg) | Ratio in weight APAP/NAC | Recommended number of intakes per day for an adult |
|---|---|---|---|---|
| 1 | 500 | 100 | 5 | 10 to 12 |
| 2 | 600 | 100 | 6 | 8 to 10 |
| 3 | 700 | 100 | 7 | 7 to 9 |
| 4 | 800 | 150 | 5,33 | 6 to 8 |
| 5 | 900 | 150 | 6 | 5 to 7 |
| 6 | 1000 | 200 | 5 | 5 to 7 |
| 7 | 1250 | 250 | 5 | 4 to 6 |
| 8 | 500 | 60 | 8,33 | 10 to 12 |
| 9 | 500 | 75 | 6,66 | 10 to 12 |
| 10 | 1000 | 150 | 6,66 | 5 to 7 |
| 11 | 1000 | 250 | 4 | 5 to 7 |
| 12 | 1250 | 300 | 4 | 4 to 6 |

The present invention concerns each dosing unit n°1 to n°12 presented in table 1.

A preferred dosing unit according to the invention comprises 1000 mg of acetaminophen and 200 mg of N-acetyl cysteine (dosing unit n°6 in table 1).

The administration of a dosing unit will be performed at least 4 times a day to an adult, preferably 5 to 7 times a day, and in particular 6 times a day.

Examples of dosing units adapted for administration to children are presented in table 2 below.

**Table 2. Examples of dosing units for children**

| Liquid formulation n° | Acetaminophen (APAP) (mg) | N-acetyl cysteine (NAC) (mg) | Ratio in weight APAP/NAC | Recommended dosing unit for a child of 20 kilograms |
|---|---|---|---|---|
| 1 | 50 | 10 | 5 | 6 intakes of 250 mg APAP per day = 1500 mg / day |
| 2 | 60 | 10 | 6 | 6 intakes of 300 mg APAP per day = 1800 mg / day |
| 3 | 70 | 10 | 7 | 6 intakes of 350 mg APAP per day = 2100 mg / day |
| 4 | 80 | 15 | 5,33 | 6 intakes of 400 mg APAP per day = 2400 mg / day |
| 5 | 90 | 15 | 6 | 5 intakes of 450 mg APAP per day = 2250 mg / day |
| 6 | 100 | 20 | 5 | 4 intakes of 500 mg per day = 2000 mg / day |
| 7 | 50 | 6 | 8,33 | 6 intakes of 250 mg APAP per day = 1500 mg / day |
| 8 | 50 | 7,5 | 6,66 | 6 intakes of 250 mg APAP per day = 1500 mg / day |
| 9 | 100 | 15 | 6,66 | 4 intakes of 500 mg APAP per day = 2000 mg / day |
| 10 | 100 | 25 | 4 | 4 intakes of 500 mg APAP per day = 2000 mg / day |

The present invention concerns each dosing unit n°1 to n°10 presented in table 2.

A preferred dosing unit according to the invention comprises 100 mg/ml of acetaminophen and 20 mg/ml of N-acetyl cysteine and is administered by doses of 5 ml (dosing unit n°6 in table 2).

The administration of a dosing unit (for example, 5 ml) will be performed at least 4 times a day to a child, preferably 5 to 7 times a day, and in particular 6 times a day.

The present invention concerns dosing units that are adapted to any suitable form for administration to a patient, in particular for oral, systemic, transdermal, nasal, ocular or rectal administration.

In a specific embodiment, the pharmaceutical formulation is an oral pharmaceutical formulation.

Oral pharmaceutical formulations include tablets, gelatin capsules, powders, granules, syrups, oral solutions or suspensions, and sublingual and buccal dosage formulations.

In a specific embodiment, the oral pharmaceutical formulation is under the form of a syrup, a tablet, a capsule or a powder.

Oral pharmaceutical formulations usually include suitable pharmaceutical carriers such as gelatin, starch, lactose, magnesium stearate, talc and gum. Oral tablets can be coated with sucrose or any other suitable substances. A syrup and a powder may contain a sweetener and/or a flavoring agent and/or a colorant.

In another specific embodiment, the pharmaceutical formulation is an injectable pharmaceutical formulation. Injectable formulations include liquid formulations adapted for subcutaneous, intravenous or intramuscular injections.

In a specific embodiment of the invention, in the dosing units, NAC is formulated as a sustained release formulation.

### Kits

The present invention also concerns a pharmaceutical kit comprising a plurality of dosing units as defined above.

Said kits may also comprise a notice for users, presenting the intended posology.

For kits intended for children, comprising liquid dosing units such as syrup, a dosing graduated cup may also be present in the kit.

The present invention also relates to a method for preventing and/or treating fever and/or pain, comprising the administration to an adult or a child in need therefore of a combination of acetaminophen and N-acetyl cysteine, wherein:
- the administered daily dose of acetaminophen is superior to 4000 mg per day for an adult, or is superior to 60 mg per weight kilo per day for a child, and
- acetaminophen and N-acetyl cysteine are administered to said adult or child according to a ratio in weight of acetaminophen / N-acetyl cysteine comprised between 1 and 10.

### EXAMPLES

Although the present invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

### Example 1. Effects of combination of APAP and NAC on animal models and healthy volunteers

The following elements have been obtained from previous animal studies and toxicity evaluation in humans.

In mice, the co-administration of APAP and NAC (in a ratio of 1) has been tested, effects on liver damages and glutathione levels were measured and compared with APAP alone and NAC alone. Administration of APAP with NAC clearly prevents APAP toxicity in mice. Authors propose that combinations of APAP and NAC be available over the counter for human beings, for decreasing APAP toxicity and therefore preventing consequences of APAP overdose (Owumi *et al*., 2015).

It has been shown in a rat model that the efficacy of acetaminophen for treating pain is not decreased when acetaminophen is combined with NAC. The analgesic effect could even be increased in presence of N-acetyl cysteine. Furthermore, no adverse effect on liver enzymes and oxidative stress was noted (Nakhaee *et al*., 2021). Authors conclude that these results may justify a reduction of acetaminophen dose for treating pain and/or fever when combined with NAC.

Finally, a randomized double-blind trial in 24 healthy subjects has been realized (Pickering *et al*., 2018). APAP was given at a daily dose of 4000 mg in four intakes of 1000 mg, with NAC at a daily dose of 600 mg (2 intakes of 300 mg) or with placebo, for a period of four days. Thermal pain tests were realized to establish the effects of APAP. This study showed that APAP, at a daily dose of 4000 mg, diminishes glutathione stock in whole blood, but this diminution is efficiently counteracted on by oral administration of NAC. Furthermore, the antinociceptive property of APAP is not influenced by NAC.

### Example 2. Animal studies: considered protocol

An experimental protocol for testing 6 administrations per day of acetaminophen, instead of 4, is implemented on mice. Animals are all males, aged of 8 to 12 weeks, this age corresponding to the young adult stage.

Analgesia effects and hepatic toxicity are followed during this experiment.

The response to pain is compared for the 4 following groups:
- Acetaminophen + NAC group: 20 animals
- Acetaminophen high dose group: 20 animals
- Acetaminophen group: 20 animals
- NAC group: 20 animals

The potential toxicity of the treatments will be quantified by imaging and molecular analysis.

The hot plate test (Bioseb^{®}) is the reference test of the animal's sensitivity to pain, allowing to maintain said pain at the minimum, since the animal is removed from the plate as soon as it shows any sign of discomfort. The reaction time is an indication of the animal's resistance to pain and is classically used to evaluate the efficacy of analgesic substances.

The dosing of liver enzymes is a classic process for assessing liver function. The glutathione assay allows the evaluation of the efficacy of NAC as a precursor of glutathione.

The monitoring of liver function by MRI allows direct observation of the liver parenchyma as well as of bile ducts and hepatic vessels, and thus to have a macroscopic view of the possible treatment induced changes. Note that non-clinically significant hepatic cytolysis is expected in the "acetaminophen alone" groups at the tested dose.

### Procedure A: Measure of the pain after drug administration

Mice are accustomed for 2 consecutive days (D-3 and D-2) to be put on the hot plate for 10 minutes, adapted to the temperature of the room.

At D-1, a blood sample is taken to assess the basal state of liver function in each group. MRI is also performed in the same goal of control.

At D0, drugs are administered by gavage, during 24 hours, according to the following therapeutic scheme:
- Acetaminophen + NAC group: 100 mg/kg paracetamol + 100 mg/kg NAC every 4 hours (6 administrations in total)
- High dose acetaminophen group: 100 mg/kg every 4 hours (6 administrations in total)
- Acetaminophen group "conventional regimen": 100 mg/kg paracetamol every 6 hours (4 administrations in total)
- NAC group: 100 mg/kg NAC every 4 hours (6 administrations in total).

One hour before drug administration, mice are subjected to the plate heated to 55.1°C. The latency until the first signs of discomfort (paw licking, paw lifting or jumping) is recorded. Animals are removed from the plate as soon as they show these signs to minimize this discomfort time. A hot plate test is performed every 6 hours until 23 hours post-treatment (5 hot plate tests in total at H-1, H5, H11, H17 and H23).

At the end of the protocol, a new blood sample is taken in each group to evaluate the state of the liver function following the administration of the different treatments. An MRI is performed for the same purpose. Then animals are euthanazied.

### Procedure B: Magnetic resonance imaging

All mice undergo MRI (7T Bruker BioSpin) basally (D-1) and at the end of the protocol under deep anesthesia (1 to 3% isofluorane mixed with 100% oxygen), including T1 FLASH and T2 RARE sequences. All images are obtained in the axial plane and synchronized to respiration to minimize motion artifacts.

The 4 groups of animals previously described are used:
- Acetaminophen + NAC experimental group: n=20
- NAC only control group: n=20
- Acetaminophen only, conventional regimen: n=20
- High doses acetaminophen: n=20

Statistical analyses are performed with GraphPad Prism software (version 9).

### Example 3. Pharmaco-epidemiology: data search in VigiBase^{®}, the WHO pharmacovigilance database

Reports of adverse events suspected to be caused by paracetamol were searched in VigiBase^{®}. They were divided in 2 groups depending on the co-administration of NAC.

Seriousness (Table 3) and seriousness criteria (Table 4) were compared between the two groups and the median daily dose of administered NAC was searched.

**Table 3. Observed seriousness in patients after administration of paracetamol, with or without NAC**

| | **Paracetamol + NAC** | | **Paracetamol without NAC** | |
|---|---|---|---|---|
| Serious | Count | Percentage | Count | Percentage |
| Yes | 376 | 23.4 | 57 331 | 33.6 |
| No | 1 065 | 66.2 | 73 160 | 42.8 |
| Unknown | 168 | 10.4 | 40 212 | 23.6 |

**Table 4. Seriousness criteria observed in patients after administration of paracetamol, with or without NAC**

| | **Paracetamol + NAC** | | **Paracetamol without NAC** | |
|---|---|---|---|---|
| **Seriousness criteria** | **Count** | **Percentage** | **Count** | **Percentage** |
| Death | 47 | 2.9 | 17 248 | 10.1 |
| Life threatening | 54 | 3.4 | 4057 | 2.4 |
| Caused/prolonged hospitalization | 266 | 16.5 | 27 640 | 16.2 |
| Disabling/incapacitating | 6 | 0.4 | 592 | 0.3 |
| Congenital anomaly/birth defect | 0 | 0 | 269 | 0.2 |
| Other medically important condition | 139 | 8.6 | 21 188 | 12.4 |

The daily dose of NAC was available in 159 reports. The median daily dose was 600 mg [IQR (Interquartile range): 600-2010].

These data suggest a protective effect of NAC in preventing fatality after acetaminophen related toxicity. In particular, it has been observed that 2.9% death is reported in patients treated with acetaminophen + NAC, as compared to 10.1% death in patients treated with acetaminophen only.

### REFERENCES

### PATENTS

US 2008/0139654
WO 02/078627
FR 2 744 917

### ARTICLES AND REVIEWS

Ramlawi M, Marti C, Sarasin F. Intoxication aiguë au paracétamol. Rev Med Suisse 2013;9(394):1478-82.
Nakhaee S, Dastjerdi M, Roumi H, Mehrpour O, Farrokhfall K. N-acetylcysteine dosedependently improves the analgesic effect of acetaminophen on the rat hot plate test. BMC Pharmacology and Toxicology 2021;22(1):4.
Owumi SE, Andrus JP, Herzenberg LA, Herzenberg LA. Co-administration of N-Acetylcysteine and Acetaminophen Efficiently Blocks Acetaminophen Toxicity. Drug Dev Res 2015;76(5):251-8.
Pickering G, Macian N, Papet I, Dualé C, Coudert C, Pereira B. N-acetylcysteine prevents glutathione decrease and does not interfere with paracetamol antinociceptive effect at therapeutic dosage: a randomized double-blind controlled trial in healthy subjects. Fundam Clin Pharmacol 2019;33(3):303-11.

## Claims

1. Combination of acetaminophen and N-acetyl cysteine for its use in preventing and/or treating fever and/or pain, wherein:
- the administered daily dose of acetaminophen is superior to 4000 mg per day for an adult, or is superior to 60 mg per weight kilo per day for a child, and
- acetaminophen and N-acetyl cysteine are administered to said adult or child according to a ratio in weight of acetaminophen / N-acetyl cysteine comprised between 1 and 10.

2. Combination for its use according to claim 1, wherein the administered daily dose of acetaminophen is comprised between 5000 mg and 10000 mg per day for an adult.

3. Combination for its use according to claim 1, wherein the administered daily dose of acetaminophen is comprised between 75 mg and 120 mg per weight kilo per day, for a child.

4. Combination for its use according to anyone of claims 1 to 3, wherein said ratio in weight is comprised between 4 and 10, preferentially is comprised between 5 to 9.

5. Combination for its use according to anyone of claims 1 to 4, wherein acetaminophen and N-acetyl cysteine of the combination are administered simultaneously or sequentially.

6. Combination for its use according to anyone of claims 1 to 5, wherein the combination is administered for a period of at least one day.

7. Dosing unit comprising, in a pharmaceutical formulation, a combination of acetaminophen and N-acetyl cysteine for its use according to anyone of claims 1 to 6.

8. Dosing unit according to claim 7, adapted for an adult, comprising 500 to 1000 mg of acetaminophen and 50 to 1000 mg of N-acetyl cystein.

9. Dosing unit according to claim 8, comprising 1000 mg of acetaminophen and 200 mg of N-acetyl cystein.

10. Dosing unit according to claim 8 or 9, wherein a dosing unit is administered at least five times a day to an adult.

11. Dosing unit according to anyone of claims 7 to 10, wherein the pharmaceutical formulation is an oral pharmaceutical formulation.

12. Dosing unit according to claim 11, wherein said oral pharmaceutical formulation is under the form of a syrup, a tablet, a capsule or a powder.

13. Dosing unit according to anyone of claims 7 to 10, wherein the pharmaceutical formulation is an injectable pharmaceutical formulation.

14. Pharmaceutical kit comprising a plurality of dosing units according to anyone of claims 7 to 13.
